# EUROPÄISCHE PATENTSCHRIFT

(11) **EP 4 426 228 B1**
(45) Veröffentlichungstag und Bekanntmachung des Hinweises auf die Patenterteilung: **24.09.2025**
(21) Anmeldenummer: 22843694.5
(22) Anmeldetag: 21.12.2022
(51) Int. Cl.: A61B 90/30, A61F 9/008, A61F 9/007, A61B 18/26

(54) **VORRICHTUNG ZUM BELEUCHTEN EINES BEHANDLUNGSORTES, INSBESONDERE IN DER OPHTHALMOLOGIE**
DEVICE FOR ILLUMINATING A TREATMENT SITE, IN PARTICULAR IN OPHTHALMOLOGY
DISPOSITIF D'ÉCLAIRAGE D'UN SITE DE TRAITEMENT, EN PARTICULIER EN OPHTALMOLOGIE

(30) Priorität: 21.12.2021 DE 102021134143
(43) Veröffentlichungstag der Anmeldung: 11.09.2024
(73) Patentinhaber: A.R.C. Laser GmbH, 90411 Nürnberg (DE)
(72) Erfinder: THYZEL, Reinhardt, 90542 Eckental (DE)
(74) Vertreter: Meissner Bolte Nürnberg
(86) Internationale Anmeldenummer: PCT/EP2022/087310
(87) Internationale Veröffentlichungsnummer: WO 2023/118347

(56) Entgegenhaltungen:
- WO-A1-2009/116969
- US-A1- 2010 160 903

## Beschreibung

Die Erfindung betrifft eine Vorrichtung zum Beleuchten eines Behandlungsortes, insbesondere eines Behandlungsortes in einem menschlichen oder tierischen Körper, insbesondere in einem Auge.

In der Ophthalmologie (Augenheilkunde) sind Behandlungsinstrumente oder Applikatoren bekannt, in denen durch Laserpulse ein Plasma erzeugt wird (im Folgenden auch "Laserplasma-Applikatoren" genannt). Der Applikator ist als in das Auge einzuführende Hohlnadel ausgebildet. An dem Nadelende des Applikators ist im Nadelinnenraum ein Target, üblicherweise aus Titan (Ti) oder einer Titanlegierung, und in der Nähe zum Target eine Öffnung in der, meist auch aus Titan (Ti) oder einer Titanlegierung gebildeten, Nadelwandung vorgesehen sind. Die Hohlnadel weist im Nadelinnenraum ferner eine dem Target mit einem freien Ende gegenüberliegende Laserlichtfaser (oder: Lichtleitfaser, optische Faser) auf zum Leiten und Richten der Laserpulse auf das Target auf. Die Laserpulse werden von einem Laser erzeugt und an einem Ende in die Laserlichtfaser eingekoppelt und treten aus dem anderen, freien Ende der Lichtleitfaser wieder aus und treffen dann auf das Target. Diese Laserpulse sind von solcher Intensität (Leistung) und Zeitdauer gewählt, dass in dem Targetmaterial ein optischer Durchbruch oder Zusammenbruch (*optical breakdown*) erzeugt wird, der zu der Ausbildung eines Plasmas oder einer Plasmawolke vor dem Target führt (laserinduziertes Plasma).

Das Nadelinnere des Applikators wird normalerweise mit einer Arbeitsflüssigkeit wie Wasser oder einer elektrolytischen Spüllösung wie BSS gefüllt, die von außen aus dem Auge durch die Öffnung und dann durch den Nadelinnenraum angesaugt wird (Aspiration mit Irrigation durch andere Irrigationsinstrumente) oder auch innerhalb der Nadel zugeführt und nach außen durch die Öffnung der Nadel geführt wird (interne Irrigation). Diese Irrigation ist notwendig, um einen Abfall des Augeninnendrucks aufgrund des Eindringens des Applikators in das Auge zu verhindern.

Das plötzliche oder sehr schnelle Aufbauen und Expandieren des laserinduzierten Plasmas führt zu einem sehr schnellen und starken Temperatur- und Druckanstieg in der Arbeitsflüssigkeit, wodurch wiederum Schockwellen (oder: Stoßwellen, Druckpulse) in der Arbeitsflüssigkeit innerhalb des Nadelinnenraumes im Bereich des Nadelendes entstehen. Diese Schockwellen treten über die Öffnung in der Nadelwandung nach außen aus und können zur Behandlung im Auge verwendet werden. In anderen Anwendungen kann auch das Plasma direkt aus der Austrittsöffnung austreten und zur Behandlung im Auge verwendet werden. In beiden Fällen ist die Öffnung in der Nadelwandung dann eine Austrittsöffnung für austretendes Plasma oder austretende Schockwellen. Es ist aber auch möglich, durch die Öffnung Gewebe herein zu saugen und mit dem Plasma oder ggf. auch den Druckpulsen zu zerstören oder stückweise abzutrennen und die Überreste durch die Nadel abzusaugen. In diesem Fall ist die Öffnung also eine Eintrittsöffnung für eintretendes Gewebe oder Material.

Die Targetoberfläche des Targets, auf die die Laserpulse treffen, wird in ihrer räumlichen Anordnung innerhalb der Nadel des Applikators im Allgemeinen einerseits teilweise in Richtung zum Austrittsende der Laserlichtfaser ausgerichtet, damit das Laserlicht möglichst steil einfällt und eine hohe Strahlungsdichte oder Intensität pro Fläche erzielt wird, und andererseits teilweise bereits in Richtung auf die Öffnung ausgerichtet, damit das Plasma oder die Druckpulse oder Stoß- oder Schockwellen eine Ausbreitungskomponente in Richtung zur Austrittsöffnung hin haben. Mit anderen Worten, die effektive, von den Laserpulsen getroffene oder bestrahlte Targetoberfläche ist im Mittel oder zumindest in Teilflächen zur Austrittsöffnung hin gerichtet. Es liegen also, von dieser effektiven Targetoberfläche aus gesehen, das freie Ende oder Austrittsende der Laserlichtfaser einerseits und die Austrittsöffnung in der Nadelwandung andererseits in einem Raumwinkelbereich von kleiner als 180° in Sichtverbindung, d.h. ein aus der Laserlichtfaser austretender Laserlichtstrahl würde ohne Plasma von der Targetoberfläche so reflektiert, dass zumindest ein Teil dieses Laserlichtstrahls aus der Austrittsöffnung austräte. Diese, immer einen Kompromiss darstellende, Ausrichtung der Targetoberfläche sowohl zur Laserlichtfaser als auch zur Austrittsöffnung hin wird beispielsweise durch eine Schrägstellung einer weitgehend flachen Targetoberfläche oder eine spezielle konvexe oder stufenförmige Form der Targetoberfläche in Kombination mit einer entsprechenden Anordnung der Austrittsöffnung sowie auch des freien Faserendes ermöglicht.

Der räumliche Abstand des freien Endes der Laserlichtfaser vom Target ist bei der gewählten Laserpulsintensität und Laserpulsdauer ausreichend groß gewählt, so dass die maximale räumliche Ausdehnung des Plasmas nicht dieses Ende der Laserlichtfaser erreichen und das empfindliche Material der Laserlichtfaser beschädigen kann.

Das Plasma entsteht kurz nach dem Beginn eines Laserpulses durch den optischen Durchbruch im Targetmaterial und hält auch noch für eine gewisse Zeit nach dem Ende eines Laserpulses an, wobei die gesamte Plasmadauer in der Regel deutlich länger ist als die Laserpulsdauer des das Plasma generierenden Laserpulses. Die Plasmadauer des laserinduzierten Plasmas ist abhängig von der Laserpulsintensität und Laserpulsdauer des Laserpulses und vom verwendeten Targetmaterial sowie auch von dem Druck bzw. dem Volumenstrom der verwendeten umgebenden Flüssigkeit.

Die zeitlichen Abstände aufeinanderfolgender Laserpulse werden nun im Allgemeinen größer als die Plasmadauer gewählt, so dass das Plasma eines Laserpulses vor dem Eintreffen des nächsten Laserpulses wieder kollabiert ist. Plasma ist nämlich spätestens bei einer Eindringtiefe, bei der eine kritische Elektronendichte erreicht ist, für elektromagnetische Strahlung unterhalb der Plasmafrequenz nicht mehr durchlässig und reflektiert die elektromagnetische Strahlung, wirkt also wie ein Spiegel für das Licht.

Das in den genannten Applikatoren verwendete Laserlicht ist üblicherweise aus dem Infrarot-Spektrum nahe dem sichtbaren Spektrum, z.B. mit einer Wellenlänge von 1064 nm bei einem Neodym-YAG-Laser, und somit aus einem Frequenzbereich unterhalb der Plasmafrequenzen der am Target auftretenden laserinduzierten Plasmen und würde deshalb das Plasma nicht zum Target durchdringen können, wenn der nächste Laserpuls bereits aus dem Laserlichtleiter austreten würde, solange noch Plasma mit einer entsprechend hohen Elektronengasdichte vor dem Target vorhanden ist.

Wenn die Laserpulsdauer der Laserpulse beispielsweise in einem Bereich von 1 ns bis 10 ns gewählt ist, liegt die Plasmadauer des Plasmas typischerweise im Bereich von 50 µs bis 200 µs, also mindestens dem 5.000-fachen der Laserpulsdauer. Um also die Abstände der Laserpulse größer als die Plasmadauer von den genannten 50 µs bis 200 µs einzustellen, wird man bei regelmäßiger Abfolge der Laserpulse und bei den genannten Laserpulsdauern von 1 ns bis 10 ns eine Pulsfrequenz (Wiederholungsrate) der Laserpulse von weniger als 20 Hz für einen Laserpulsabstand von mehr als einer Plasmadauer von 50 µs und weniger als 5 Hz für einen Laserpulsabstand von mehr als einer Plasmadauer von 200 µs wählen oder einstellen.

Der häufigste Anwendungsfall solcher Applikatoren mit laserinduzierter Plasmaerzeugung (im Folgenden kurz Laserplasma-Applikatoren genannt) in der Ophthalmologie ist
- das Zerstören der natürlichen Augenlinsen und Ersetzen durch künstliche Linsen zur Behebung eines Katarakts (grauer Star) oder zur Anpassung oder Korrektur der optischen Brennweite, beispielsweise bei Myopie oder Hyperopie. Solche Laserplasma-Applikatoren sind beispielsweise aus US 5,324,282 A mit einer schrägen Targetoberfläche oder US 5,906,611 A mit einer gestuften Targetoberfläche und jeweils Austrittsöffnungen am Nadelende bekannt.

Weitere Anwendungen solcher Laserplasma-Applikatoren in der Ophthalmologie sind
- die Entfernung von Epithelzellen bei der Behandlung von Sekundärkatarakten gemäß WO 2005/107665 A1 oder gemäß EP 2 683 080 B1 mit einem Target an einem geschlossenen Nadelende und einer seitlichen Austrittsöffnung in der Nadelwandung,
- das Abtragen von Melanozyten von der Stroma zum Verändern der Augenfarbe gemäß WO 2018/069013 A1*,*
- das Behandeln des Trabekelwerkes mit Plasma für die Glaukomtherapie gemäß EP 3 628 281 A1 mit einer zentralen Öffnung im Nadelende des Applikators sowie
- das Abtragen von Glaskörpermaterial in der Vitrektomie gemäß der EP 3 626 213 A1 mit einer seitlichen länglichen Austrittsöffnung in der Nadelwandung und gemäß der noch nicht veröffentlichten DE 10 2020 115 885 mit einem Applikator mit gekrümmter Nadel.

Die Beleuchtung des Behandlungsortes im Auge für die behandelnde Person wird durch externe Lampen oder Mikroskoplicht vorgesehen.

Aus der US 2010/160903 A1 ist ein Verfahren zum Verringern einer unerwünschten endoluminalen Struktur, die an einer Behandlungsstelle in einem Säugetier-Behandlungssubjekt vorhanden ist, bekannt, wobei die endoluminale Struktur einen Gefäßverschluss, einen Biofilm oder eine andere unerwünschte biologische Struktur umfasst. Das Verfahren umfasst eine Anwendung mechanischer Stoßwellen auf die endoluminale Struktur; wobei die mechanischen Stoßwellen von der endoluminalen Struktur absorbiert werden, welche dadurch zerkleinert werden kann. Es ist eine Beleuchtungseinrichtung zur Beleuchtung des zu behandelnden Zielbereichs vorgesehen. Die Beleuchtungseinrichtung kann eine Beleuchtungsfaser mit einem proximalen Lichteingangsende, das mit einer Lichtquelle in Verbindung steht, und mit einem distalen Lichtausgangsende, das in der Nähe der Behandlungsstelle angeordnet werden kann, um die Behandlungsstelle zu beleuchten, umfassen. Ein Behandlungszyklus umfasst in sequentieller Ausführung eine Beleuchtung mit Inspektion, gefolgt von einer Stoßwellenbehandlung und einer anschließenden Spülung und/oder Absaugung.

Die WO 2009/116969 A1 offenbart ein Beleuchtungssystem mit einem Arthroskop, einem Endoskop oder einem anderen geeigneten chirurgischen Instrument und eine anbringbare Kanüle aus einem transparenten oder halbtransparenten Material, das in der Lage ist, Licht vom proximalen Ende der Kanüle zum distalen Ende der Kanüle zu leiten und dadurch ein Operationsfeld zu beleuchten. Das Operationsfeld wird durch Komponenten beleuchtet, die keinen Platz beanspruchen, der sonst für die Optik des Arthroskops verwendet werden könnte. Die Kanüle besteht aus einem sterilisierbaren Polymer, das als Wellenleiter fungiert.

Eine Aufgabe der Erfindung ist es, eine neue Vorrichtung und ein neues Verfahren jeweils zum Beleuchten eines Behandlungsortes, insbesondere eines Behandlungsortes in einem menschlichen oder tierischen Körper, insbesondere in einem Auge, anzugeben. Insbesondere soll eine *in situ* Beleuchtung für den zu behandelnden Bereich im Auge geschaffen werden.

Diese Aufgabe wird gemäß der Erfindung insbesondere gelöst durch den Gegenstand des unabhängigen Patentanspruchs 1. Ausgestaltungen und Weiterbildungen ergeben sich aus den abhängigen Patentansprüchen sowie aus der nachfolgenden Beschreibung und den Zeichnungen.

Die beanspruchbaren Merkmalskombinationen und Gegenstände gemäß der Erfindung sind nicht auf die gewählte Fassung und die gewählten Rückbeziehungen der Patentansprüche beschränkt. Jedes Merkmal in den Patentansprüchen, auch unabhängig von deren Rückbeziehungen, kann in einer beliebigen Kombination mit einem oder mehreren anderen Merkmal(en) in den Patentansprüchen beansprucht werden. Außerdem kann jedes Merkmal, das in der Beschreibung oder Zeichnung beschrieben oder offenbart ist, für sich, unabhängig oder losgelöst von dem Zusammenhang, in dem es steht, allein oder in jeglicher Kombination mit einem oder mehreren anderen Merkmalen, das oder die in den Patentansprüchen oder in der Beschreibung oder Zeichnung beschrieben oder offenbart ist oder sind, beansprucht werden.

Im Folgenden werden Ausführungsbeispiele gemäß der Erfindung beschrieben. Dabei wird auch auf die Zeichnungen Bezug genommen, in deren
- FIG 1: eine Hohlnadel eines Applikators im vorderen Bereich mit ihrem distalen-Ende in einem Längsschnitt,
- FIG 2: die Hohlnadel gemäß FIG 1 in einer Gesamtansicht von unten,
- FIG 3: die Hohlnadel gemäß FIG 1 im vorderen Bereich mit ihrem distalen Ende im Betrieb des Applikators beim Erzeugen von Plasma mittels eines Laserstrahls in einem Längsschnitt,
- FIG 4: die Hohlnadel gemäß FIG 1 und FIG 3 im vorderen Bereich mit ihrem distalen Ende beim Beleuchten mittels Beleuchtungslicht im Betrieb des Applikators in einem Längsschnitt,
- FIG 5: die Hohlnadel gemäß FIG 4 in einer Innenansicht auf das distale Ende,
- FIG 6: eine Koppeleinrichtung eines Applikators, insbesondere nach FIG 1 bis 4, zum Koppeln eines Lichtleiters an eine Laserlichtquelle und eine Beleuchtungslichtquelle und
- FIG 7: eine Vorrichtung für die Vitrektomie mit einem Applikator und einer Zentraleinheit mit Laserlichtquelle und Beleuchtungslichtquelle sowie Koppeleinrichtung
jeweils schematisch dargestellt sind. Sofern nicht anderweitig explizit beschrieben, werden gleiche oder funktionsgleiche Elemente in den Figuren mit den gleichen Bezugszeichen bezeichnet. Ferner sind die Figuren nicht zwingend maßstabsgetreu.

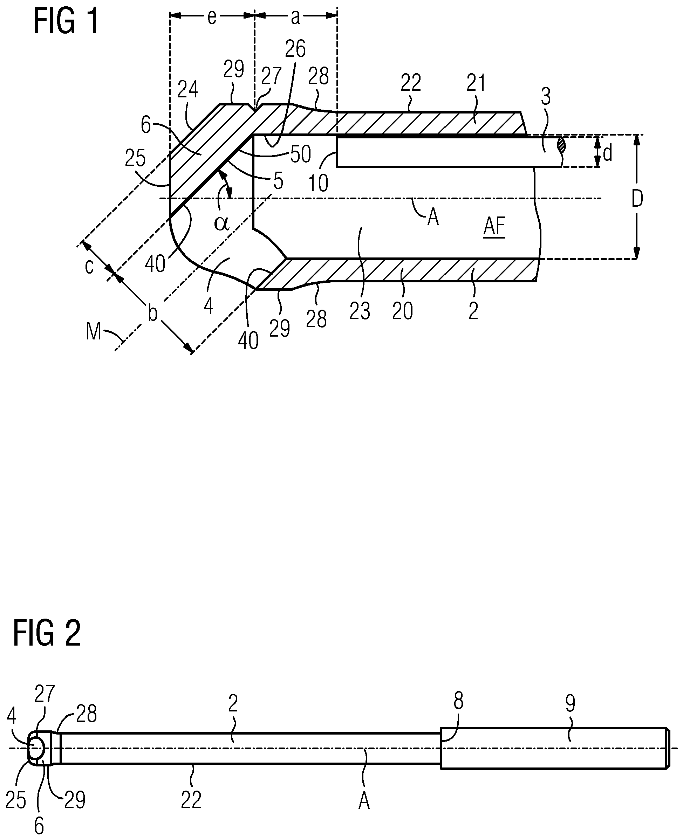

In dem Ausführungsbeispiel gemäß FIG 1 und FIG 2 umfasst ein Applikator 2 eine Hohlnadel 20, deren Nadelwandung 21 einen Hohlraum oder Nadelinnenraum 23 der Hohlnadel 20 umschließt und die entlang einer Applikatorachse (oder allgemein bei auch gekrümmtem Verlauf der Hohlnadel 20 eine Applikatorzentrallinie) A verläuft, und einen Lichtleiter 3, der im Nadelinnenraum 23 der Hohlnadel 20 verläuft.

Ein Nadelinnendurchmesser D des Nadelinnenraumes 23 ist typischerweise aus einem Bereich von 0,1 mm bis 0,9 mm gewählt oder, gestützt auf die medizinische Fachterminologie, im Bereich von 20G bis 25G (G: Gauge).

An einem distalen Ende 6 der Hohlnadel 20 ist im Nadelinnenraum 23 ein Target 5 angeordnet mit einer Targetoberfläche 50. Ein freies Ende 10 mit einer optischen Austrittsfläche des Lichtleiters 3 liegt der Targetoberfläche 50 unter einem Abstand a beabstandet gegenüber. Der Abstand a beträgt insbesondere zwischen 0,7 mm und 1,2 mm, insbesondere etwa 0,9.

Der Lichtleiter 3 ist vorzugweise eine Lichtleitfaser oder optische Faser mit einem Durchmesser d, der typischerweise aus einem Bereich von 150 µm bis 400 µm, insbesondere 283 µm, gewählt ist.

Ferner ist in der Nadelwandung 20 im Bereich des distalen Endes 6 eine Öffnung 4 ausgebildet mit einem Öffnungsdurchmesser b und einer Mittelachse M und einer die Öffnung 4 umgebenden Öffnungsinnenfläche 4. Durch die Öffnung 4 ist der Innenraum der Hohlnadel 20 mit dem Außenraum verbunden. Im Betrieb des Applikators während der Behandlung ist der Außenraum ein Behandlungsbereich, in dem menschlichen oder tierischen Körper, vorzugsweise in einem Auge, in den die Hohlnadel 20 mit ihrem distalen Ende 6 eingeführt wurde. Die Mittelachse M der Öffnung 4 ist im dargestellten Ausführungsbeispiel gegen die Applikatorachse A um einen Neigungswinkel α geneigt, der 45° beträgt, allgemein aber bevorzugt aus einem Bereich von 30° bis 60° gewählt ist. Der Öffnungsdurchmesser b ist insbesondere aus einem Bereich von 0,2 mm bis 1,4 mm gewählt, insbesondere etwa 0,7 mm.

Die Hohlnadel 20 und ihr Nadelinnenraum 23 kann aus einem massiven Rohling durch Einführen eines speziellen ersten Bohrers mit einem dem gewünschten Nadelinnendurchmesser D des Nadelinnenraumes 23 entsprechenden Bohreraußendurchmesser axial zur Applikatorachse A ausgehend von einem zum distalen Ende 6 entgegengesetzten proximalen Ende 8 erzeugt werden, wobei die Nadelwandung 21 stehenbleibt mit einer überwiegend zylindrischen Außenfläche 22 und wobei sich beim Bohren der Nadelwandung 21 am distalen Ende 6 zunächst eine konische Innenfläche ergibt, die die Bohrerspitze abbildet, beispielsweise mit einem Öffnungswinkel von 60°.

Die Öffnung 4 kann nun durch Einführen eines zweiten Bohrers durch die Nadelwandung 21 im Bereich des distalen Endes 6 in Bohrrichtung entlang der Mittelachse M mit einem dem Öffnungsdurchmesser b entsprechenden Bohreraußendurchmesser erzeugt werden. Dieses anschließende Bohren der Öffnung 4 erzeugt eine (weitere) zylindrische Innenfläche der Nadelwandung 21, die sich aus der Öffnungsinnenfläche 40 der Öffnung 4 und einer dann ebenfalls mit diesem zweiten Bohrer erzeugte, auf einer Zylinderfläche um die Mittelachse M mit dem Zylinderdurchmesser b verlaufenden Targetoberfläche 50 des Targets 5 fortsetzt. Die zylindrische Targetoberfläche 50 des Targets ist dann wie die Mittelachse M unter dem Neigungswinkel α zur Applikatorachse A geneigt. Die Targetoberfläche 50 kann auch zusätzlich einen konischen Teilbereich aufweisen, der noch von dem ersten Bohrvorgang oder dem ersten Bohrer stehenbleibt.

Das Target 5 ist als, in FIG 1 mittlerer, Teilbereich des nach den beiden Bohrschritten am distalen Ende 6 der Hohlnadel 20 stehenbleibenden und stärkeren Wandbereichs der Nadelwandung 21 mit der axialen Länge e und der größeren Wandstärke c, die auch der Wandstärke des Targets 5 entspricht, gebildet. Dieser stärkere Wandbereich der Nadelwandung 21 ist auf der Außenseite der Nadelwandung 21 beispielsweise von einer Abschrägung 24, die konisch oder als Fase ausgebildet sein kann, und an der Stirnseite von einer senkrecht zur Applikatorachse A gerichteten flachen Stirnfläche 25 begrenzt.

Die größere Wandstärke c des Targets 5 hinter der Targetoberfläche 50 kann, ohne Beschränkung der Allgemeinheit, insbesondere zwischen 0,1 mm und 0,5 mm liegen und dient u.a. dazu, dem Targetmaterialabtrag durch die Laserpulse LP Rechnung zu tragen. Außerhalb des Targets 5 oder im Bereich der Außenfläche 22 kann die Nadelwandung 21 eine geringere Wandstärke beispielsweise um die 0,1 mm aufweisen.

Es ist aber auch, insbesondere durch andere Fertigungsschritte, möglich und im Rahmen der Erfindung sinnvoll, eine andere Form der Targetoberfläche 50 zu erzeugen, beispielsweise zumindest teilweise eine konische Form, insbesondere mit der Applikatorachse A als Symmetrieachse, oder eine flache Form oder auch eine Stufenform oder eine im Längsschnitt konkave oder konvexe Form.

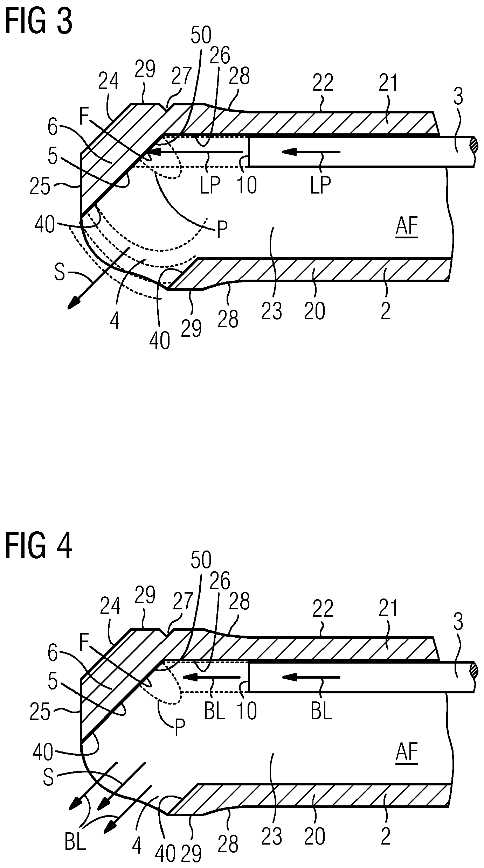

Ferner ist, wie in FIG 1 (und FIG 3 und 4) dargestellt, die Nadelwandung 21 im Bereich des distalen Endes 6 auf einen größeren Außendurchmesser vergrößert, im Allgemeinen schon im Rohling vor dem Erzeugen des Nadelinnenraumes 23 im ersten Bohrschritt. Die zylindrischer Außenfläche 22 der Nadelwandung 21 mit einem vorgegebenen Außendurchmesser geht zum distalen Ende 6 hin über einen, beispielsweise konkav gekrümmten, Übergangsbereich 28 in eine zylindrischer Außenfläche 29 der Nadelwandung 21 mit einem vorgegebenen Außendurchmesser, der größer ist als der Außendurchmesser der Außenfläche 22, über. Dies kann beispielsweise auch dazu dienen, einen sog. Sleeve als Abdichtung vor einem Abstreifen nach vorne zu bewahren.

In dem axialen Bereich der Nadelwandung 21, in dem innen die zweite Bohrung für die Öffnung 4 oder die Targetoberfläche 50 in die zylindrische Innenfläche der Nadelwandung 21 mit dem Innendurchmesser D übergeht, ist überdies an der Außenseite der Nadelwandung 21 eine Einkerbung oder Markierung 7 vorgesehen., die an der gegenüberliegenden Seite teilweise von der Öffnung 4 vorgesehen ist und die Lage der Öffnung 4 markiert.

Anhand von FIG 3 wird nun das Wirkungsprinzip und die Funktion des Applikators gemäß FIG 1 und 2 zur laserinduzierten Plasmaerzeugung erläutert. Eine laserinduzierte Plasmaerzeugung ist an sich bekannt, insbesondere bei den eingangs erwähnten Applikatoren mit laserinduzierter Plasmaerzeugung.

Mittels des Lichtleiters 3 wird Laserlicht (oder: Laserstrahlung) zum distalen Ende 6 der Hohlnadel 20 übertragen, in der Regel in Form von Laserpulsen LP. Die Laserpulse LP treten aus dem auf das Target 5 gerichteten freien Ende 8 des Lichtleiters 3 aus und treffen in einem Fokalbereich F der Laserpulse LP auf die Targetoberfläche 50. Die Leistungsflächendichten oder Energieflächendichten und die Pulsdauern der Laserpulse LP werden nun so gewählt, dass in dem vorgegebenen Targetmaterial des Targets 5 an der Targetoberfläche 50 oberflächennah ein optischer Durchbruch stattfindet und Plasma P in den Nadelinnenraum 23 vor der Targetoberfläche 50 austritt.

Bei einem Durchmesser d des Lichtleiters 3 von 270 µm resultiert, zur Berechnung der Energieflächendichte oder Leistungsflächendichte, eine Querschnittsfläche am freien Ende 10 von ungefähr (0,27 mm)² π = 0,23 mm².

Die Pulsdauern der Laserpulse LP werden typischerweise und abhängig von der Anwendung des Applikators 2 aus einem Bereich von 1 ns bis 10 ns gewählt. Die Energie pro Laserpuls LP wird vorzugsweise aus einem Bereich von 2 mJ bis 15 mJ eingestellt. Bei solchen Laserpulsen LP entsteht Plasma P mit einer Plasmadauer typischerweise im Bereich von 50 µs bis 200 µs, also mindestens dem 5.000-fachen der Laserpulsdauer. D.h. der in FIG 3 gezeigte Zustand ist nur ein Anfangs- oder Induzierungszustand für eine kurze Zeit, nämlich die Pulsdauer des Laserpulses LP, und danach herrscht erst einmal über eine viel längere Zeit ein Zustand ohne Laserpuls LP, aber mit dem Plasma P, also wie in FIG 3, nur ohne den Laserpuls LP.

Das Laserlicht für die Laserpulse LP ist üblicherweise aus dem Infrarot-Spektrum nahe dem sichtbaren Spektrum, z.B. mit einer Wellenlänge von 1064 nm, wie von einem Neodym-YAG-Laser geliefert. Solche Laserstrahlung ist aus einem Frequenzbereich weit unterhalb der Plasmafrequenzen der am Target 5 auftretenden laserinduzierten Plasmas P.

Da das aufgebaute Plasma P die Laserstrahlung reflektiert, werden die zeitlichen Abstände der Laserpulse LP größer als die Plasmadauer von den genannten 50 µs bis 200 µs eingestellt oder wird eine Pulsfrequenz oder Pulswiederholungsrate der Laserpulse von weniger als 20 Hz für einen Laserpulsabstand von mehr als einer Plasmadauer von 50 µs und weniger als 5 Hz für einen Laserpulsabstand von mehr als einer Plasmadauer von 200 µs eingestellt.

Bei dem optischen Durchbruch werden durch die Hochleistungs-Laserpulse LP an der Targetoberfläche 50 in dem bestrahlten Fokalbereich F nicht nur (quasi) freie Elektronen aus dem Leitungsenergieband (Leitungselektronen), sondern auch durch einen Lawineneffekt gebundene Elektronen aus den Elektronenhüllen der Atome des Targetmaterialgefüges herausgelöst und dadurch Targetatome zusätzlich ionisiert. Die während der Laserpulsdauer übertragene Laserpulsenergie ist im Allgemeinen nur so groß gewählt, dass aus dem Target 5 im Wesentlichen nur Elektronen und keine ionisierten Targetatome in das Plasma P herausgelöst werden.

Das Targetmaterial des Targets 5 ist deshalb bevorzugt ein Metall oder eine Metalllegierung aufgrund der bereits vorhandenen Leitungselektronen und hohen Elektronendichte und der im Vergleich zu Dielektrika dadurch wesentlich schneller oder bei wesentlich niedrigeren Laserleistungen und geringere Materialzerstörungen stattfindenden optischen Durchbrüche. Ein bevorzugtes Material für das Target 5 ist Titan (Ti) oder einer Titanlegierung, vorzugsweise aus oder mit Titan (Ti), wobei das Titan beispielsweise Ti-Grade 4 sein kann. Im dargestellten Ausführungsbeispiel ist das Target 5 einstückig mit der Nadelwandung 21 gebildet, die somit aus dem gleichen Material, insbesondere Metall oder Metalllegierung, vorzugsweise aus Titan (Ti) oder einer Titanlegierung, besteht.

Das aus dem Target 5 ausgetretene Plasma P expandiert und breitet sich mit hoher Geschwindigkeit von der Targetoberfläche 50 weg nach innen in den Nadelinnenraum 23 aus und nimmt ungefähr die Form einer projektil- oder domförmigen und, von der Targetoberfläche 50 aus gesehen, konvexen Plasmawolke an, wie in FIG 3 und 4 skizziert ist. Diese ausgehend von dem Fokalbereich F gesehen konvexe oder quasi fokussierte Form der Plasmawolke P ergibt sich auch aus der konvexen und quasi fokussierenden, insbesondere zylindrischen und/oder konischen, Form der Targetoberfläche 50 im Fokalbereich F.

Im Betrieb des Applikators 2 bei einer Augenbehandlung ist der Nadelinnenraum 23 normalerweise mit einer Arbeits- oder Irrigationsflüssigkeit AF wie Wasser oder einer elektrolytischen Spüllösung wie BSS gefüllt, die von außen aus dem Auge durch die Öffnung 4 und den Nadelinnenraum 23 angesaugt wird und durch ein Irrigationsinstrument zugeführt wird, oder auch durch den Nadelinnenraum 23 zugeführt und nach außen durch die Öffnung 4 austritt. Diese Irrigation ist notwendig, um einen Abfall des Augeninnendrucks aufgrund des Eindringens des Applikators in das Auge zu verhindern.

Diese Irrigations- oder Arbeitsflüssigkeit AF dämpft und bremst nun die Ausdehnung des Plasmas P im Nadelinnenraum 23 ("löscht" das Plasma). Zugleich führt aber die plötzliche Druckerhöhung und der sehr starke Temperaturanstieg durch das laserinduzierte Plasma P zu der Ausbildung von Schockwellen S (oder:Druckpulsen, Stoßwellen), die sich in der Flüssigkeit AF im Nadelinnenraum 23 ausbreiten und durch die Öffnung 4 zumindest teilweise nach außen austreten. Je nach Anwendung werden auch hohe Pulsraten und damit Schockwellenraten gewählt und/oder können die nächsten Laserpulse LP möglichst nahe am Ende der Plasmadauer oder bereits während des Kollabierens der vorhergehenden Laserpulse LP gesendet werden, um das Plasma "aufrechtzuerhalten".

Diese durch das laserinduzierte Plasma erzeugten Schockwellen S sind nun in der Regel das eigentliche Behandlungsmedium für die Behandlung.

Bevorzugte Anwendungsfälle des Applikators 2 mit laserinduzierter Plasmaerzeugung und der damit erzeugten Schockwellen in der Ophthalmologie sind die schon eingangs erwähnten Augenlinseneingriffe wie das Zerstören der natürlichen Augenlinsen und Ersetzen durch künstliche Linsen zur Behebung eines Katarakts (grauer Star) oder zur Anpassung oder Korrektur der optischen Brennweite, beispielsweise bei Myopie oder Hyperopie, die Entfernung von Epithelzellen bei der Behandlung von Sekundärkatarakten und das Abtragen von Melanozyten von der Stroma zum Verändern der Augenfarbe.

Eine weitere (direkte) Anwendung des Plasmas P des Applikators 2 ist möglich beim Öffnen des Trabekelwerkes für die Glaukomtherapie und beim Abtragen von Glaskörpermaterial in der Vitrektomie.

Ferner sind auch medizinische Anwendungen außerhalb der Ophthalmologie möglich z.B. in Lithotripsie zum Zertrümmern von Gallen- oder Nierensteinen und auch Anwendungen außerhalb der Medizin, z.B. bei der Feinrestaurierung von Kunstwerken und Architektur, z.B. Fresken.

Nachdem nun die Hauptfunktion des Applikators ausreichend beschrieben worden ist, soll sich nun der neuen Zusatzfunktion gemäß der Erfindung für den Applikator zugewandt werden, nämlich der integrierten Beleuchtung.

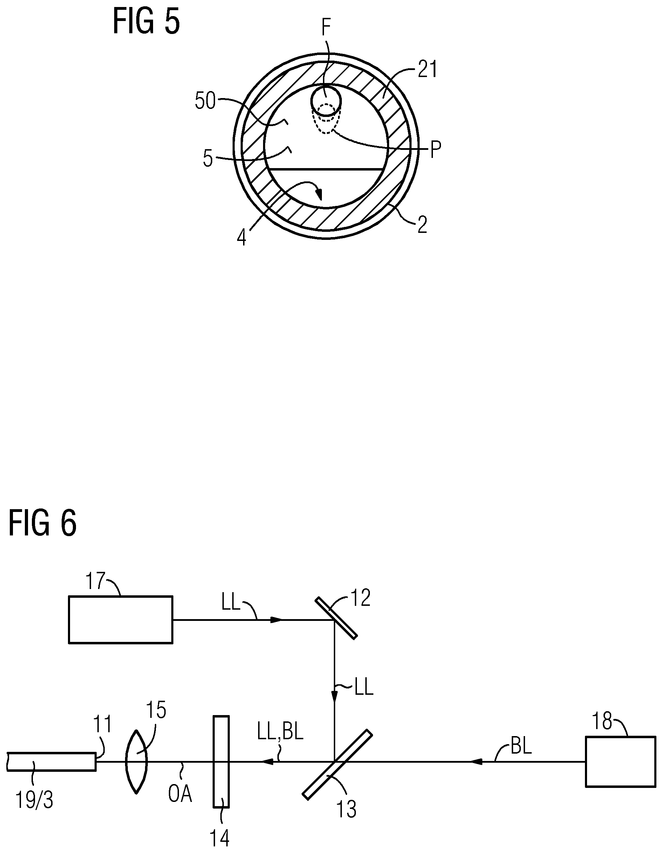

Gemäß der Erfindung allgemein und den in FIG 4 und FIG 5 gezeigten Ausführungsbeispielen im Besonderen wird in dem Applikator eine *in situ* Beleuchtung oder Illumination für den Ort vorgesehen, an dem die Behandlung stattfindet. Dazu wird Beleuchtungslicht aus der Hohlnadel an den Behandlungsort geschickt und zwar durch denselben Lichtleiter, durch den die Laserpulse zur Erzeugung des Plasmas geschickt werden.

Dass dies physikalisch möglich ist, hätte die Fachwelt einschließlich des auf diesem Gebiet seit Jahrzehnten sehr erfahrenen Erfinders der vorliegenden Erfindung, nicht für möglich gehalten. Beleuchtungslicht aus dem Lichtleiter folgt demselben Strahlengang wie die Laserpulse hin zum Target und trifft damit zwangsläufig auf das von den Laserpulsen erzeugte und deutlich länger anhaltende Plasma vor der Targetoberfläche. Da das optische Frequenzspektrum von Beleuchtungslicht, insbesondere auch Weißlicht, unterhalb der Plasmafrequenz liegt, wird das Beleuchtungslicht von dem Plasma wie von einem Spiegel reflektiert und kann deshalb das Plasma nicht durchdringen und nicht zu der Öffnung gelangen und sich somit auch nicht zum Behandlungsort ausbreiten können.

Dennoch haben technische Überlegungen und Versuche des Erfinders gezeigt, dass ein beträchtlicher Teil des Beleuchtungslichts aus dem Lichtleiter trotz des Plasmas dennoch zur Öffnung in der Nadelwandung und von dort nach draußen gelangen kann, wenn man bestimmte technische Vorgaben für den Applikator einstellt und einhält.

Die der Erfindung zugrundeliegende Überlegung besteht darin, das Plasma und die Innenwandung der Hohlnadel am distalen Ende als Reflexions- oder Spiegelflächen für das Beleuchtungslicht zu verwenden und so das Beleuchtungslicht durch Mehrfachreflexionen am Plasma und der Nadelinnenwandung durch die Öffnung, insbesondere die Öffnung 4, nach außen zu führen.

Die Laserpulse werden hinsichtlich ihrer Leistungsdichtewerte und Pulsdauern so eingestellt und der Abstand a des freien Endes 10 des Lichtleiters 3 von der Targetoberfläche 50 groß genug gewählt, dass das durch die Laserpulse erzeugte Plasma weniger weit von der Targetoberfläche 50 weg expandiert als der Abstand a oder Zwischenraum zwischen der Targetoberfläche 50 und dem freien Ende 10 des Lichtleiters 3 und damit weit genug weg bleibt von dem freien Ende 10 des Lichtleiters 3, wie in FIG 4 dargestellt. Die vorne angegebenen Werte für die Laserpulse LP und den Abstand a erfüllen bereits diese Bedingungen. Dadurch ist das Plasma immer von dem freien Ende des Lichtleiters beabstandet und der Anteil des nach hinten von der Öffnung 4 weg reflektierten Beleuchtungslichts BL wird klein gehalten.

Ferner wurde durch Messungen die Beobachtung gewonnen, dass das Plasma ausgehend von dem Fokalbereich des Laserlichtes aus dem Lichtleiter auf der Targetoberfläche eine von der Targetoberfläche aus gesehen konvexe und langgestreckte Ausdehnung oder Gestalt annimmt, wie in FIG 3 und 4 schematisch dargestellt ist. Dadurch liegt im Strahlengang des aus dem Lichtleiter austretenden Beleuchtungslichts eine konvexe Plasmaspiegelfläche, die zwar in der Projektion den gesamten Strahlquerschnitt des Beleuchtungslichts ausfüllt, die aber auch aufgrund ihrer konvexen langgestreckten Form das Beleuchtungslicht BL über einen wesentlichen Teil des Strahlquerschnitts mit einer Komponente in Richtung der Ausbreitungsrichtung des Beleuchtungslichts beim Austreten aus dem Lichtleiter weiterreflektiert.

Schließlich ist die Innenwandung der Hohlnadel, also die Innenfläche 26 der Nadelwandung 21 und die Öffnungsinnenfläche 40 zumindest im Bereich des distalen Endes 6 lichtreflektierend oder spiegelnd auszubilden, so dass das von dem Plasma P zur Innenfläche 26 hin reflektierte Beleuchtungslicht BL von der Innenfläche 26 der Nadelwandung 21 erneut reflektiert wird und dann entweder bereits an dem Plasma P außen vorbei oder durch Mehrfachreflexionen zwischen Innenfläche 26 der Nadelwandung und Plasma schließlich zur Öffnung in der Nadelwandung gelangt und dort austreten kann.

Die Spiegeloberfläche an der Innenfläche 26 und 40 (sowie auch 50) der Nadelwandung 21 (und 6) ist bevorzugt sehr glatt ausgebildet, beispielsweise mit einer Rauhtiefe von unter 10 µm, und wird insbesondere durch die mit sehr hohen Drehzahlen von 6000 bis 8000 U/min drehenden und unter hohem Vorschubdruck bewegten feinen Bohrer zum Erzeugen des Nadelinnenraums 23 sowie der Öffnung 4 erzeugt. Diese Mehrfachreflexionen hin zur Öffnung 4 werden unterstützt durch die konvexe, insbesondere zylindrische, Form der Innenflächen 26 und 40, die das Beleuchtungslicht BL zur Öffnung 4 oder deren Mittelachse M hin leitet oder konzentriert.

Die FIG 4 und FIG 5 zeigen nun sehr schematisch und skizzenhaft die Situation mit einem noch vorhandenen Plasma P nach dem Abschalten des Laserpulses LP und zugleich dem nun aus dem Lichtleiter 3 am freien Ende 10 in den Nadelinnenraum 23 eintretenden Beleuchtungslicht BL.

Das Beleuchtungslicht BL ist Licht aus dem sichtbaren Spektrum mit einem vorgegebenen oder einstellbaren Frequenzspektrum, insbesondere Weißlicht mit einem breiten Frequenzspektrum aus dem sichtbaren Bereich, insbesondere 400 nm bis 800 nm oder 420 nm bis 780 nm.

Nun ist zwar das Beleuchtungslicht BL, ebenso wie zuvor das Laserlicht der Laserpulse LP, in Richtung auf den Fokalbereich F an der Targetoberfläche 50 gerichtet, erreicht die Targetoberfläche 50 aber nicht, sondern trifft zuvor auf das Plasma P vor der Targetoberfläche 50. Vom Plasma P wird das Beleuchtungslicht BL nun reflektiert, da die Lichtfrequenzen des Beleuchtungslichts BL unterhalb der Plasmafrequenz liegen. Nach der Reflexion am Plasma P trifft das Beleuchtungslicht BL sodann auf die Innenwandung, insbesondere die Innenfläche 26 oder 40 der Nadelwandung 21 und wird dort wieder nach innen reflektiert, trifft dann wieder auf das Plasma P oder verläuft an dem Plasma P vorbei zur Öffnung 4. Durch diese Mehrfachreflexionen erreicht das Beleuchtungslicht BL trotz des vorhandenen Plasmas letztendlich die Öffnung 4, an der das Beleuchtungslicht BL dann austritt, wie in FIG 4 gezeigt. Die Öffnung 4 ist auch relativ groß gewählt, wodurch mehr von dem mehrfach reflektiertes Beleuchtungslicht BL aufgefangen werden kann und aus der Öffnung 4 austreten kann.

Der Anteil des durch Mehrfachreflexionen von dem freien Ende des Lichtleiters zur Öffnung 4 gelangenden Beleuchtungslichts wird durch die vergleichsweise kleine Fläche des Fokalbereichs F und damit des Plasmas F im Vergleich zu der gesamten Querschnittsfläche D² π der Hohlnadel und durch die oben beschriebene konvexe Gestaltung der Nadelinnenwandung in Bezug auf die Mittelachse M der Öffnung 4 und die Applikatorachse A weiter befördert . Auch die teilweise Ausrichtung der Targetoberfläche 50 zur Öffnung 4 hin verstärkt den zur Öffnung 4 mehrfach reflektierten Anteil des Beleuchtungslichts BL.

Das Beleuchtungslicht BL kann insbesondere durchgehend leuchten oder aus dem Lichtleiter 3 austreten, wenn es vom Nutzer eingeschaltet ist, also auch während der Laserpulse LP eingeschaltet sein.

Die Beleuchtung direkt am Behandlungsort im Auge hat insbesondere den Vorteil für die Patienten, dass das vergleichsweise grelle und unangenehme Mikroskoplicht beim Eingriff heruntergedimmt werden kann.

FIG 6 zeigt nun eine Koppeleinrichtung zum Koppeln eines vom distalen Ende 10 abgewandten oder entgegengesetzten proximalen Endes 11 des Lichtleiters 3 (oder eines zwischengeschalteten Verbindungslichtleiters 19) mit sowohl einer Laserlichtquelle 17, die die Laserpulse LP aussendet, beispielsweise eine Nd:YAG Laserquelle, als auch einer Beleuchtungslichtquelle 18, die das Beleuchtungslicht BL aussendet. Die Laserpulse LP der Laserlichtquelle 17 werden über einen Umlenkspiegel 12 einem Strahlkombinierer 13 zugeführt, der mit einem teildurchlässigen oder IR refelektierenden und sichtbares Licht transmittierenden und unter 45° angeordneten Spiegel gebildet ist, und von diesem um 90° auf eine optische Achse OA umgelenkt Um 90° versetzt wird dem Strahlkombinierer 13 das Beleuchtungslicht BL der Beleuchtungslichtquelle 18 zugeführt und passiert diesen geradlinig und verläuft dann ebenfalls auf der optischen Achse OA. Die Laserpulse LP und das Beleuchtungslicht BL werden nun über einen Shutter 14 und eine Sammellinse 15 in das Ende 11 des Lichtleiters 3 oder 19 eingespeist. Die Beleuchtungslichtquelle 18 kann mit mehreren Dioden gebildet sein, beispielsweise RGB, um das Frequenzspektrum oder auch eine Farbtemperatur des Beleuchtungslichts BL einstellen zu können.

Anstelle einer einzigen Lichtleitfaser kann in nicht dargestellten Ausführungsformen der Lichtleiter 3 auch mehrere, insbesondere nebeneinander geführte, Lichtleitfasern umfassen, wobei das Laserlicht und das Beleuchtungslicht jeweils durch eine dieser mehreren Lichtleitfasern geleitet werden. In weiteren Ausgestaltungen ist es auch möglich, dass eine oder mehrere Lichtleitfasern des Lichtleiters für das Laserlicht vorgesehen sind, und dass eine oder mehrere Lichtleitfasern für das Beleuchtungslicht vorgesehen sind.

Grundsätzlich kann in einer alternativen, nicht dargestellten Ausführungsform ein Lichtleiter oder eine Lichtleitfaser für das Beleuchtungslicht auch näher an der Öffnung oder entfernt vom Plasmabereich enden, so dass das Beleuchtungslicht durch die Öffnung nach außen gelangen kann, ohne vorher auf das Plasma zu treffen.

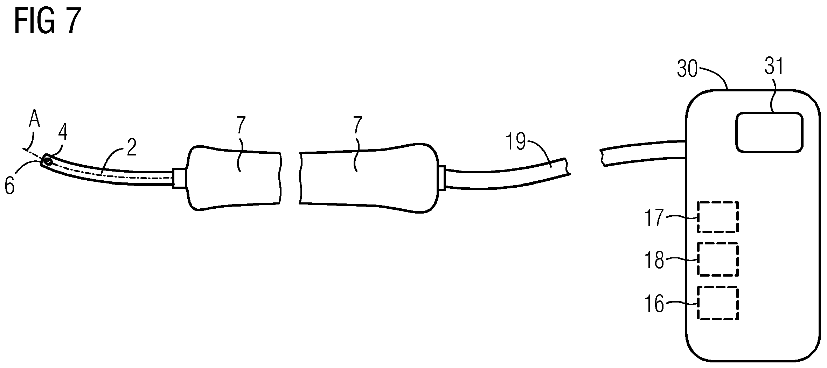

Eine gesamte ophthalmologische Vorrichtung, insbesondere für die Vitrektomie, ist in FIG 7 gezeigt. Der Applikator 2 ist hier als Vitrektom und die Hohlnadel 20 für einen besseren Zugang im Auge wenigstens abschnittsweise gekrümmt ausgebildet, insbesondere im Endabschnitt bis zum distalen Ende 6, an dem die Öffnung 4 vorgesehen ist. Die Hohlnadel 20 ist mit einem Griffteil 7 und ihr Lichtleiter 3 optisch mit einem Lichtleiter 19 (einem Lichtleitkabel) gekoppelt, der am anderen Ende mit einer Zentraleinheit 30 optisch verbunden ist. Die Zentraleinheit (oder: Schnittstelle) 16 weist eine Laserlichtquelle 17 und eine Beleuchtungslichtquelle 18 auf sowie eine Koppeleinrichtung zum optischen Koppeln der Laserlichtquelle 17 und der Beleuchtungslichtquelle 18 jeweils mit dem Lichtleiter 19 und damit dem Vitrektom oder Applikator 2.

Es ist ferner, insbesondere in der Zentraleinheit 30 eine Absaugeinheit 16 vorgesehen, die an den Nadelinnenraum 23 der Hohlnadel 20 angeschlossen ist und einen Unterdruck von beispielsweise von 0,2 bar bis 0,4 bar unter Atmosphärendruck zum Ein- oder Ansaugen von Glaskörpermaterial in den Nadelinnenraum 23 zur Ablation des Glaskörpers mittels des Plasmas P und zum Absaugen der abgetragenen Glaskörperstückchen. Zum Ausgleich für das abgeführte Glaskörpermaterial kann dem Auge eine Ersatzflüssigkeit wie Silikonöl zugeführt werden.

Eine entsprechende Vorrichtung kann auch mit einem anderen Applikator kombiniert werden, beispielsweise einem Applikator nach den FIG 1 bis 5 und/oder für andere Anwendungen vorgesehen sein. Die Vorrichtung kann eine Steuer- oder Kontrolleinrichtung zur Steuerung oder Kontrolle der Laserlichtquelle und der Beleuchtungslichtquelle aufweisen. Die Steuereinrichtung kann eine oder mehrere elektronische Schaltungen und/oder Prozessoren umfassen. Die die Steuereinrichtung, insbesondere die elektronische Schaltung und/oder der oder die Prozessoren ist/sind dazu eingerichtet, zumindest die Laserlichtquelle und die Beleuchtungslichtquelle zur Ausführung bzw. Durchführung einer Behandlung anzusteuern.

Die Vorrichtung kann weitere Komponenten umfassen, beispielsweise außer oder anstatt der Ansaugeinheit auch eine Irrigationsvorrichtung zum Einbringen von Spül- oder Ersatzflüssigkeit ins Auge.

### Bezugszeichenliste

- 2: Applikator
- 3: Lichtleiter
- 4: Öffnung
- 6: distales Ende
- 7: Griffteil
- 8: proximales Ende
- 9: Zwischenteil
- 10: freies Ende
- 11: Ende des Lichtleiters
- 12: Spiegel
- 13: Strahlkombinierer
- 14: Shutter
- 15: Linse
- 16: Absaugeinheit
- 17: Laserlichtquelle
- 18: Beleuchtungslichtquelle
- 19: Lichtleiter
- 20: Hohlnadel
- 21: Nadelwandung
- 22: Außenfläche
- 24: Abschrägung
- 25: Stirnfläche
- 26: Innenfläche
- 27: Ringnut
- 28: Übergangsbereich
- 29: Außenfläche
- 30: Zentraleinheit
- 31: Benutzerschnittstelle
- 40: Öffnungsinnenwand
- 50: Targetoberfläche
- a: Abstand Lichtleiter zu Target
- b: Durchmesser Öffnung
- c: Wandstärke
- d: Durchmesser Lichtleiter
- e: axiale Länge
- A: Applikatorachse
- BL: Beleuchtungslicht
- D: Innendurchmesser Hohlnadel
- F: Fokusbereich
- LL: Laserlicht
- M: Mittelachse Öffnung
- OA: optische Achse
- P: Plasma
- α: Neigungswinkel

## Patentansprüche

1. Vorrichtung zum Beleuchten eines Behandlungsortes, insbesondere eines Behandlungsortes in einem menschlichen oder tierischen Körper, insbesondere in einem Auge, umfassend
a) einen Applikator (2) und eine
b) Beleuchtungslichtquelle (18) zum Erzeugen von Beleuchtungslicht (BL),
c) wobei der Applikator eine Hohlnadel (20) mit einem distalen Ende (6) und mit einem Target (5) an dem distalen Ende (6) sowie mit einer Öffnung (4) an dem distalen Ende (6) aufweist,
d) wobei der Applikator einen in der Hohlnadel (20) zu dem distalen Ende (6) geführten Lichtleiter (8) mit einem zum Target (5) hin orientierten freien Ende (10) aufweist,
e) wobei über den Lichtleiter (8) Laserpulse (LP) übertragen werden oder übertragbar sind, die am freien Ende (10) aus dem Lichtleiter (3) austreten und auf das Target (5) treffen und vor einer Targetoberfläche (50) des Targets (5) ein Plasma erzeugen (P),
f) wobei sich im Bereich an der Öffnung (4) der zu beleuchtende Behandlungsort befindet und das Plasma zur direkten oder indirekten Behandlung am Behandlungsort vorgesehen ist,
g) wobei die Beleuchtungslichtquelle (18) mit dem Lichtleiter (3) des Applikators optisch gekoppelt oder koppelbar ist, derart, dass das Beleuchtungslicht (BL) der Beleuchtungslichtquelle (18) über den Lichtleiter (3) übertragen wird und am freien Ende (10) aus dem Lichtleiter (3) austritt,
**dadurch gekennzeichnet, dass**
h) eine Innenfläche (26, 40) der Hohlnadel (20) am distalen Ende (6) als Spiegelfläche (26, 40) für das Beleuchtungslicht (BL) ausgebildet ist, derart, dass das Beleuchtungslicht (BL) nach dem Austritt aus dem Lichtleiter (3) von dieser Spiegelfläche (26, 40) und zumindest teilweise unter einer oder mehrerer Zwischenreflexionen von dem Plasma (P) zu der Öffnung (4) reflektiert wird.

2. Vorrichtung nach Anspruch 1, bei der die Spiegelfläche (26, 40) zumindest um das Target (5) und um die Öffnung (4) angeordnet ist.

3. Vorrichtung nach Anspruch 1 oder Anspruch 2 mit einer Laserlichtquelle (17) zum Erzeugen der Laserpulse und mit einer optischen Kopplungseinrichtung (16) zur unmittelbaren oder mittelbaren optischen Kopplung des Lichtleiters (8) an die Laserlichtquelle (17) und die Beleuchtungslichtquelle (18).

4. Vorrichtung nach einem der vorhergehenden Ansprüche, bei der das Beleuchtungslicht Weißlicht ist und/oder aus dem optischen Frequenzspektrum unterhalb der Plasmafrequenz des Plasmas gewählt ist, so dass das Plasma als Reflexions- oder Spiegelfläche für das Beleuchtungslicht wirkt.

5. Vorrichtung nach einem der vorhergehenden Ansprüche, bei der der Abstand (a) des freien Endes (10) des Lichtleiters (3) von der Targetoberfläche (50) so groß gewählt ist, dass das durch die Laserpulse erzeugte Plasma vom freien Ende (10) des Lichtleiters (3 ) beabstandet ist, wobei dieser Abstand (a) insbesondere einem Wert zwischen 0,7 mm und 1,2 mm, insbesondere etwa 0,9 mm, entspricht.

6. Vorrichtung nach einem der vorhergehenden Ansprüche, bei der die Öffnung (4) eine Mittelachse (M) aufweist, die vorzugsweise gegen eine Applikatorachse (A) der Hohlnadelwandung (20) um einen Neigungswinkel α geneigt ist, der bevorzugt aus einem Bereich von 30° bis 60°, vorzugsweise 45°, gewählt ist, und eine zylindrische Öffnungswandung (40) aufweist.

7. Vorrichtung nach einem der vorhergehenden Ansprüche, wobei ein Nadelinnenraum (23) der Hohlnadel (2) mittels eines entlang der Applikatorachse (A) eingeführten ersten Bohrers erzeugt ist und die Öffnung (4) mittels eines entlang der Mittelachse (M) eingeführten zweiten Bohrers erzeugt ist, wobei vorzugsweise mittels hoher Bohrerdrehzahlen, beispielsweise von 6000 bis 8000 U/min, glatte Spiegelflächen für das Beleuchtungslicht erzeugt werden.

8. Vorrichtung nach Anspruch 3, wobei die Laserlichtquelle (17) derart eingerichtet ist, dass diese im Betrieb die Laserpulse erzeugt, wobei die Laserpulse einer Pulsdauer aus einem Bereich von 1 ns bis 10 ns haben und/oder eine Pulsenergie aus einem Bereich von 2 mJ bis 15 mJ, haben und/oder der Abstand zweier Laserpulse größer gewählt ist als die Plasmadauer des Plasmas, wobei die Plasmadauer insbesondere in einem Bereich von 50 µs bis 200 µs liegt.

9. Vorrichtung nach nach Anspruch 8, wobei die Laserlichtquelle (17) ferner derart eingerichtet ist, dass bei deren Betrieb eine Beleuchtungsdauer des Beleuchtungslichts größer ist als die Zeitdauer von mehreren Laserpulsen hintereinander und/oder das Beleuchtungslicht kontinuierlich eingekoppelt wird.

## Claims

1. Device for illuminating a treatment site, in particular a treatment site in a human or animal body, in particular in an eye, comprising
a) an applicator (2) and a
b) illumination light source (18) for generating illumination light (BL),
c) wherein the applicator comprises a hollow needle (20) with a distal end (6) and with a target (5) at the distal end (6) and with an opening (4) at the distal end (6),
d) wherein the applicator has a light guide (8) guided in the hollow needle (20) to the distal end (6) with a free end (10) oriented towards the target (5),
e) wherein laser pulses (LP) are transmitted or can be transmitted via the light guide (8), which emerge from the light guide (3) at the free end (10) and strike the target (5) and generate a plasma (P) in front of a target surface (50) of the target (5),
f) wherein the treatment site to be illuminated is located in the region at the opening (4) and the plasma is intended for direct or indirect treatment at the treatment site,
g) wherein the illumination light source (18) is optically coupled or couplable to the light guide (3) of the applicator in such a way that the illumination light (BL) of the illumination light source (18) is transmitted via the light guide (3) and emerges from the light guide (3) at the free end (10),
**characterised in that**
h) an inner surface (26, 40) of the hollow needle (20) at the distal end (6) is designed as a mirror surface (26, 40) for the illumination light (BL) in such a way that the illumination light (BL) after exiting the light guide (3) is reflected by this mirror surface (26, 40) and at least partially reflected by one or more intermediate reflections from the plasma (P) to the opening (4).

2. Device according to claim 1, wherein the mirror surface (26, 40) is arranged at least around the target (5) and around the opening (4).

3. Device according to claim 1 or claim 2, with a laser light source (17) for generating the laser pulses and with an optical coupling device (16) for direct or indirect optical coupling of the light guide (8) to the laser light source (17) and the illumination light source (18).

4. Device according to one of the preceding claims, wherein the illumination light is white light and/or is selected from the optical frequency spectrum below the plasma frequency of the plasma, so that the plasma acts as a reflection or mirror surface for the illumination light.

5. Device according to one of the preceding claims, wherein the distance (a) of the free end (10) of the light guide (3) from the target surface (50) is selected to be such that the plasma generated by the laser pulses is spaced apart from the free end (10) of the light guide (3), wherein this distance (a) corresponds in particular to a value between 0.7 mm and 1.2 mm, in particular approximately 0.9 mm.

6. Device according to one of the preceding claims, wherein the opening (4) has a central axis (M) which is preferably inclined relative to an applicator axis (A) of the hollow needle wall (20) by an angle of inclination α which is preferably selected from a range of 30° to 60°, preferably 45°, and has a cylindrical opening wall (40).

7. Device according to one of the preceding claims, wherein a needle interior (23) of the hollow needle (2) is produced by means of a first drill introduced along the applicator axis (A) and the opening (4) is produced by means of a second drill introduced along the central axis (M), wherein smooth mirror surfaces for the illumination light are preferably produced by means of high drill rotation speeds, for example from 6000 to 8000 rpm.

8. Device according to claim 3, wherein the laser light source (17) is designed such that, during operation, it generates laser pulses, wherein the laser pulses have a pulse duration in a range from 1 ns to 10 ns and/or a pulse energy in a range from 2 mJ to 15 mJ, and/or the distance between two laser pulses is selected to be greater than the plasma duration of the plasma, wherein the plasma duration is in particular in a range from 50 µs to 200 µs.

9. Device according to claim 8, wherein the laser light source (17) is further arranged such that, during operation, an illumination duration of the illumination light is greater than the time duration of several laser pulses in succession and/or the illumination light is continuously coupled in.

## Revendications

1. Dispositif pour éclairer un site de traitement, en particulier un site de traitement dans un corps humain ou animal, en particulier dans un œil, comprenant
a) un applicateur (2) et une
b) source de lumière d'éclairage (18) pour générer une lumière d'éclairage (BL),
c) l'applicateur comportant une aiguille creuse (20) avec une extrémité distale (6) et une cible (5) à l'extrémité distale (6) ainsi qu'une ouverture (4) à l'extrémité distale (6),
d) l'applicateur comportant un guide de lumière (8) guidé dans l'aiguille creuse (20) vers l'extrémité distale (6) et comportant une extrémité libre (10) orientée vers la cible (5),
e) des impulsions laser (LP) étant transmises ou pouvant être transmises par le conducteur optique (8), lesquelles impulsions sortent du conducteur optique (3) à l'extrémité libre (10) et rencontrent la cible (5) et génèrent un plasma (P) devant une surface cible (50) de la cible (5),
f) le site de traitement à éclairer se trouvant dans la zone de l'ouverture (4) et le plasma étant prévu pour un traitement direct ou indirect au niveau du site de traitement,
g) la source de lumière d'éclairage (18) étant couplée optiquement ou pouvant être couplée au guide de lumière (3) de l'applicateur, de telle sorte que la lumière d'éclairage (BL) de la source de lumière d'éclairage (18) est transmise par le guide de lumière (3) et sort à l'extrémité libre (10) du guide de lumière (3),
**caractérisé en ce que**
h) une surface intérieure (26, 40) de l'aiguille creuse (20) à l'extrémité distale (6) est conçue comme une surface réfléchissante (26, 40) pour la lumière d'éclairage (BL), de telle sorte que la lumière d'éclairage (BL), après être sortie du guide de lumière (3), est réfléchie par cette surface réfléchissante (26, 40) et est réfléchie au moins en partie vers l'ouverture (4) par une ou plusieurs réflexions intermédiaires du plasma (P).

2. Dispositif selon la revendication 1, dans lequel la surface réfléchissante (26, 40) est disposée au moins autour de la cible (5) et autour de l'ouverture (4).

3. Dispositif selon la revendication 1 ou 2, comprenant une source de lumière laser (17) pour générer les impulsions laser et un dispositif de couplage optique (16) pour le couplage optique direct ou indirect du guide de lumière (8) à la source de lumière laser (17) et à la source de lumière d'éclairage (18).

4. Dispositif selon l'une des revendications précédentes, dans lequel la lumière d'éclairage est une lumière blanche et/ou est choisie dans le spectre optique de fréquences situé en dessous de la fréquence plasma du plasma, de sorte que le plasma agit comme surface de réflexion ou miroir pour la lumière d'éclairage.

5. Dispositif selon l'une des revendications précédentes, dans lequel la distance (a) entre l'extrémité libre (10) du guide de lumière (3) par rapport à la surface cible (50) est choisie de manière à ce que le plasma généré par les impulsions laser soit espacé de l'extrémité libre (10) du guide de lumière (3 ), cette distance (a) correspondant en particulier à une valeur comprise entre 0,7 mm et 1,2 mm, en particulier à environ 0,9 mm.

6. Dispositif selon l'une des revendications précédentes, dans lequel l'ouverture (4) présente un axe central (M) qui est incliné de préférence par rapport à un axe d'applicateur (A) de la paroi d'aiguille creuse (20) selon un angle d'inclinaα , qui est de préférence choisi dans une plage comprise entre 30° et 60°, de préférence 45°, et une paroi d'ouverture cylindrique (40).

7. Dispositif selon l'une des revendications précédentes, dans lequel un espace intérieur (23) de l'aiguille creuse (2) est réalisé au moyen d'un premier foret introduit le long de l'axe (A) de l'applicateur et l'ouverture (4) est réalisée au moyen d'un deuxième foret introduit le long de l'axe central (M), des surfaces lisses comme un miroir pour la lumière d'éclairage étant de préférence réalisées au moyen de vitesses de rotation élevées du foret, par exemple de 6000 à 8000 tr/min.

8. Dispositif selon la revendication 3, dans lequel la source de lumière laser (17) est agencée de telle sorte qu'elle génère les impulsions laser en fonctionnement, les impulsions laser ayant une durée d'impulsion comprise dans une plage de 1 ns à 10 ns et/ou une énergie d'impulsion comprise dans une plage de 2 mJ à 15 mJ, et/ou la distance entre deux impulsions laser est choisie supérieure à la durée du plasma, la durée du plasma étant en particulier comprise dans une plage de 50 µs à 200 µs.

9. Dispositif selon la revendication 8, dans lequel la source de lumière laser (17) est en outre agencée de telle sorte que, lors de son fonctionnement, une durée d'éclairage de la lumière d'éclairage est supérieure à la durée de plusieurs impulsions laser successives et/ou la lumière d'éclairage est couplée en continu.
